(19) **Europäisches Patentamt / European Patent Office / Office européen des brevets**

(11) **EP 3 180 722 B1**

(12) **EUROPEAN PATENT SPECIFICATION**

(45) Date of publication and mention
of the grant of the patent:
**11.10.2023 Bulletin 2023/41**

(21) Application number: **15774690.0**

(22) Date of filing: **11.08.2015**

(51) International Patent Classification (IPC):
**G16B 10/00** (2019.01)       **G16B 30/00** (2019.01)

(52) Cooperative Patent Classification (CPC):
**G16B 10/00; G16B 30/00**

(86) International application number:
**PCT/IB2015/056093**

(87) International publication number:
**WO 2016/024213 (18.02.2016 Gazette 2016/07)**

(54) **SYSTEMS AND METHODS FOR TRACKING AND IDENTIFYING INFECTION TRANSMISSION**

SYSTEME UND VERFAHREN ZUR VERFOLGUNG UND IDENTIFIZIERUNG VON INFEKTIONSÜBERTRAGUNG

SYSTÈMES ET PROCÉDÉS POUR SUIVRE ET IDENTIFIER LA TRANSMISSION D'UNE INFECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.08.2014 US 201462037413 P**

(43) Date of publication of application:
**21.06.2017 Bulletin 2017/25**

(73) Proprietor: **Koninklijke Philips N.V.**
**5656 AG Eindhoven (NL)**

(72) Inventors:
• **KAMALAKARAN, Sitharthan**
**5656 AE Eindhoven (NL)**
• **PAULSON, Joseph N.**
**5656 AE Eindhoven (NL)**

(74) Representative: **Philips Intellectual Property & Standards**
**High Tech Campus 52**
**5656 AG Eindhoven (NL)**

(56) References cited:
**US-A1- 2002 120 408      US-A1- 2010 169 122**
**US-A1- 2011 246 084      US-A1- 2011 280 907**

• **MARK N RAGHEB ET AL: "The mutation rate of mycobacterial repetitive unit loci in strains of M. tuberculosis from cynomolgus macaque infection", BMC GENOMICS, BIOMED CENTRAL, vol. 14, no. 1, 5 March 2013 (2013-03-05), page 145, XP021145089, ISSN: 1471-2164, DOI: 10.1186/1471-2164-14-145**
• **Christopher B Ford ET AL: "Mycobacterium tuberculosis mutation rate estimates from different lineages predict substantial differences in the emergence of drug-resistant tuberculosis", NATURE GENETICS, vol. 45, no. 7, 1 January 2013 (2013-01-01), pages 784-790, XP055352840, NEW YORK, US ISSN: 1061-4036, DOI: 10.1038/ng.2656**

**Description**

BACKGROUND

Healthcare-associated infections (HAIs) are patient-acquired infections received during healthcare treatment for conditions unrelated to the infection. A healthy patient entering a hospital for a surgical procedure to repair a hernia who subsequently develops a staph infection at the surgical site while in the recovery ward is an example of a patient-acquired HAI. HAIs in the medical literature are often referred to as nosocomial infections. According to a survey conducted by the CDC in 2011, approximately 1 out of every 25 patients hospitalized will contract an HAI. The study estimated that there were approximately 721,000 HAIs. HAIs cause or contribute to approximately 75,000 deaths each year.

Nosocomial infections can cause severe pneumonia and infections of the urinary tract, bloodstream and other parts of the body. Many types are difficult to attack with antibiotics, and antibiotic resistance is spreading to Gram-negative bacteria that can infect people outside the hospital. In the USA, the most frequent type of infection hospital-wide is pneumonia (21.8%), followed by surgical site infection (21.8%), and gastrointestinal infection (17.1%). (Magill SS, Edwards JR, Bamberg W, et al. "Multistate Point-Prevalence Survey of Health Care-Associated Infections," N Engl J Med 2014;370:1198-208.)

According to a 2009 report by the CDC, HAIs cost U.S. hospitals approximately $35 billion per year. Much of the cost is related to longer patient stays, quarantining parts of the hospital, and discovering and eradicating the source of infection. Approximately 25.6% of HAIs are believed to be caused by medical devices such as catheters and ventilators. The remaining infections are believed to be associated with surgical procedures and other sources within the hospital. There is a need to be able to track hospital-associated infections and determine the source of transmission to reduce the number of additional infections. (Scott II, R. D., "The Direct Medical Costs of Healthcare-Associated Infections in U.S. Hospitals and the Benefits of Prevention," CDC, March 2009.)

US 2002/120408 A1 (KREISWIRTH BARRY N [US] ET AL) 29 August 2002 discloses a method for identifying a source of an infection. A relatedness between a bacterial isolate and a stored isolate determined to identify the infection. A region of DNA that is sequenced for comparison may be that having a mutation rate suitably fast for performing real-time infection control.

Ragheb M N et al. ("The mutation rate of mycobacterial repetitive unit loci in strains of M. tuberculosis from cynomolgus macaque infection", BMC Genomics, vol. 14, no. 1, 5 March 2013) discloses an experimental estimate of the mycobacterium interspersed repetitive unit (MIRU) mutation rate in strains isolated from infected cynomolgus macaques using a whole-genome sequencing approach.

Ford C B et al. ("Mycobacterium tuberculosis mutation rate estimates from different lineages predict substantial differences in the emergence of drug-resistant tuberculosis", Nature Genetics, vol. 45, no. 7, 1 January 2013) discloses a significant variation in the mutation rates of the strains within each lineage of *M. tuberculosis* which contributes to the differences in the rates at which drug resistance is acquired.

However, none of said prior art discloses taking into account a mutation rate calculated by the Mean Path Lengths method.

SUMMARY OF THE INVENTION

According to an illustrative embodiment of the invention, there is provided a method according to claim 1.

According to an illustrative embodiment of the invention, there is provided a system according to claim 9.

BRIEF DESCRIPTION OF THE DRAWINGS

FIG. 1 is a block diagram of transmission vectors according to embodiments of the disclosure.
FIG. 2A is a phylogenetic tree according to an embodiment of the disclosure.
FIG. 2B is a dated phylogenetic tree according to an embodiment of the disclosure.
FIG. 3 is flow chart of a method according to an embodiment of the disclosure.
FIG. 4 is a block diagram of a system according to an embodiment of the system.
FIG. 5 a graph of growth rate curves according to an embodiment of the disclosure.
FIG. 6 is an empirical cumulative distribution function plot according to an embodiment of the disclosure.
FIG. 7 is a density plot according to an embodiment of the disclosure.
FIG. 8 is a flow chart of processes according to embodiments of the invention.

DETAILED DESCRIPTION OF PREFERRED EMBODIMENTS

The following description of certain exemplary embodiments is merely exemplary in nature and is in no way intended to limit the invention or its applications or uses. In the following detailed description of embodiments of the present systems and methods, reference is made to the accompanying drawings which form a part hereof, and in which are shown by way of illustration specific embodiments in which the described systems and methods may be practiced. These embodiments are described in sufficient detail to enable those skilled in the art to practice the presently disclosed systems and methods, and it is to be understood that other embodiments may be utilized and that structural and logical changes may be made without departing from the scope of the present system.

The following detailed description is therefore not to be taken in a limiting sense, and the scope of the present system is defined only by the appended claims. The leading digit(s) of the reference numbers in the figures herein typically correspond to the figure number, with the exception that identical components which appear in multiple figures are identified by the same reference numbers. Moreover, for the purpose of clarity, detailed descriptions of certain features will not be discussed when they would be apparent to those with skill in the art so as not to obscure the description of the present system.

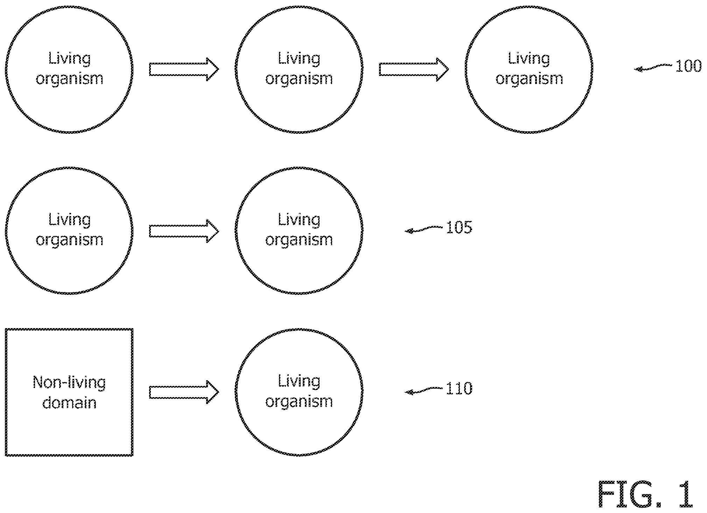

An infection may be caused by a pathogen such as bacteria, a virus, a fungus, a parasite, or other organism. Some infections may be caused by multiple types of organisms present at the same time. An infection may spread by a variety of transmission vectors. Example transmission vectors are illustrated in Figure 1. In some instances, an infection may be transmitted between two living organisms. For example, a doctor may treat a first patient with an infection. If the doctor does not thoroughly disinfect his hands, he may transmit the infection from the first patient to a second patient. This example may correspond to transmission vector 100. An infected organism may also directly transmit an infection to another organism. For example, an infected person may sneeze or cough in the vicinity of another person who inhales the pathogen and subsequently becomes infected. This example may correspond to transmission vector 105. In other instances, an infection may be transmitted to a living organism from a non-living domain. A non-living domain may be a surface such as table or a sink counter. A non-living domain may also be a piece of medical equipment such as a dialysis machine or an endoscope. For example, a patient may contract an infection when an open wound on the patient contacts a non-sterile examining table. This example is illustrated as transmission vector 110.

Determining the transmission vector (e.g., how a patient was infected), may allow an infection to be traced back to its source. The source may then be eliminated to prevent further transmission of infection. For example, if the source is a patient, the patient may be quarantined to avoid infecting other patients until the infection can be cleared with treatment (e.g., antibiotics). In one example, if the source is a piece of equipment, the equipment may be sterilized or replaced with new equipment. In a hospital setting, the ability to track an infection back to its source may reduce the incidence of HAIs and their associated costs. It may also reduce the time and costs of unnecessarily treating areas with disinfectants that are not linked to HAIs.

When an infection, such as an HAI, is detected, samples may be collected by medical staff from patients, surfaces, food, equipment, or other suspected sources. Samples may include tissue, blood, water, swabs of surfaces. The samples may then be processed to isolate the pathogen causing the infection from other materials in the sample. The infection isolate may then be analyzed by a variety of methods. The analysis may determine the pathogen type, species, drug resistance, and/or other properties. While this analysis may assist with determining effective treatments, it may not adequately identify the transmission vector.

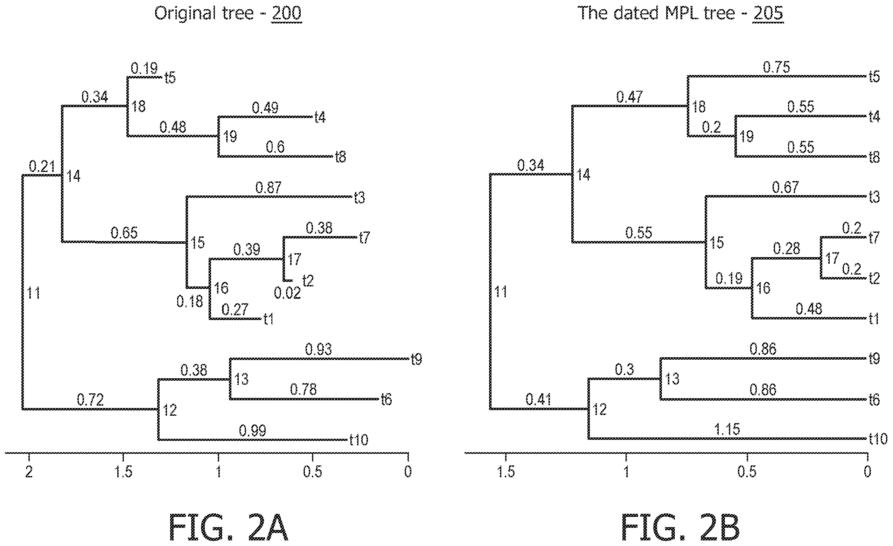

By collecting an infection isolate from an infected organism or non-living domain and analyzing its genetic sequence, the transmission vector of the infection by using phylogenetic methods may be determined. An infection isolate is a component of the sample that includes genetic information from a pathogen. Phylogenetics is the study of evolutionary relationships between organisms. Such relationships are often represented as weighted graphs such as trees. An example of a phylogenetic tree is shown in Figures 2A-B. Phylogenetic methods analyze all or a portion of a genetic sequence of an organism. By determining an evolutionary history of an infection, an infection may be traced back to its source. The evolutionary history may provide an understanding of how different incidents of an infection are or are not related. For example, the sequences of infection isolates from multiple infected patients may be compared. It may be determined when in time the patients were infected. It may also be determined that one or more of the patients were infected by a different source.

Multiple phylogenetic methods exist, including methods based on evolutionary distances, parsimonious, and maximum likelihoods. Distances based methods are where an evolutionary distance is calculated between each organism. The evolutionary distance is calculated based on the degree of similarity between genetic sequences of organisms. One such method for determining evolutionary distances is called the Jukes-Cantor (Evolution of protein molecules In Mammalian protein metabolism, Vol. III (1969), pp. 21-132 by T. H. Jukes, C. R. Cantor edited by M. N. Munro) method where the transition from any particular nucleotide in the genome to another, i.e. transitions or transversions, can occur with the same probability:

$$Q = \begin{bmatrix} -\dfrac{3\mu}{4} & \dfrac{\mu}{4} & \dfrac{\mu}{4} & \dfrac{\mu}{4} \\ \dfrac{\mu}{4} & -\dfrac{3\mu}{4} & \dfrac{\mu}{4} & \dfrac{\mu}{4} \\ \dfrac{\mu}{4} & \dfrac{\mu}{4} & -\dfrac{3\mu}{4} & \dfrac{\mu}{4} \\ \dfrac{\mu}{4} & \dfrac{\mu}{4} & \dfrac{\mu}{4} & -\dfrac{3\mu}{4} \end{bmatrix} \qquad \text{Equation 1}$$

In Equation 1, above, the instantaneous rate matrix Q represents the rates of change between a pair of nucleotides per instant of time. P - the probability transition matrix is given as

$$p(t) = e^{Qt} \qquad \text{Equation 2}$$

As a result, the evolutionary distance between any two organisms under this model is simply:

$$d_{ab} = -\frac{3}{4}\ln\left(1 - \frac{4}{3}p\right) \qquad \text{Equation 3}$$

Where p is the number of sites along the single nucleotide polymorphisms (SNPs)/DNA that differ between the sequences. The distance goes to infinity as p approaches the equilibrium value (75% of sites differ). This simple model, however, does not take into account the biological consideration that transitions (purine to purine (a-g) or pyrimidine to pyrimidine (t-c)) and transversions (purine to pyrimidine or vice-versa) occur at different rates. Another distance model, the Kimura 2-parameter model (Kimura, Motoo. "A simple method for estimating evolutionary rates of base substitutions through comparative studies of nucleotide sequences." Journal of molecular evolution 16.2 (1980): 111-120), attempts to correct for this. In this case:

$$d = -\frac{1}{2}\ln\left[(1 - 2p - q)\left(sqrt(1 - 2q)\right)\right] \qquad \text{Equation 4}$$

For p (proportion of transitions) and q (proportion of transversions).

Once infection isolate sequences have been compared to determine their evolutionary distances, rates of evolution may be determined. The evolutionary distances and relationships between infection isolates from samples may then be plotted in graphical form, such as a tree plot. Neighbor Joining (Saitou N, Nei M. "The neighbor-joining method: a new method for reconstructing phylogenetic trees." Molecular Biology and Evolution, volume 4, issue 4, pp. 406-425, July 1987) is one method of building unrooted trees. The method corrects for unequal evolutionary rates between sequences by first finding a pair of neighboring leaves i and j which have the same parent node k. That is, leaves i and j may be pathogens that evolved from a common pathogen k. Leaves i and j may then be removed from the list of leaf nodes and k is added to the current list of nodes, and node distances are recalculated. This algorithm is an example of a greedy "minimum evolution" algorithm.

Using the tree representation of many infection isolate sequence samples, the relative timing of one infection to another infection is estimated. Without loss of generality, a method called Mean Path Lengths (MPL) is used (Britton, Tom, et al. "Phylogenetic dating with confidence intervals using mean path lengths." Molecular phylogenetics and evolution 24.1 (2002): 58-65). The MPL method estimates the age of a node with the mean of the distances from this node to all leaves descending from it. Under the assumption of a similar molecular clock, that is, a rate of evolution, standard-errors of the estimated node ages can be computed. Using this method, mutation rates may be calculated for the different infection isolates.

A source of infection may be determined based on the replication rate. Pathogens may replicate at different rates in different environments. Different environments that may have reservoirs of pathogens that replicate at different rates may include but are not limited to blood, saliva, food, surgical tables, sinks, toilets, and bed linens. The replication rate may correlate with the mutation rate. Replication rate, total replication, mutation rate, total nucleotide change and other metrics that represent a measure of phylogenetic relationship, phylogenetic status, reproductive or replication history of samples may be used in addition or as substitute to the metrics described herein. As such, any phylogenetic metric, measure, indicator, or predictor is within the scope of the present invention, and is hereinafter referred to as a phylogenetic metric. Typically, the mutation rate may be lower for pathogens that replicate at a lower rate. If the replication rate of a pathogen is known for an environment, it may be determined that an infection isolate originated from that environment based on the calculated mutation rate. For example, a pathogen may replicate more quickly in a fluidic environment such as blood than in a dry environment such as the surface of a furniture. If an infection isolate taken from a patient exhibits a high mutation rate compared to a reference infection isolate, it may be determined that the patient transmitted the infection from another living organism. This determination may allow hospital staff to focus their effort of infection containment on other patients rather than testing equipment for contamination. This may allow the hospital staff to reduce the time to determining the source of infection and eliminate the cost of unnecessarily disinfecting equipment.

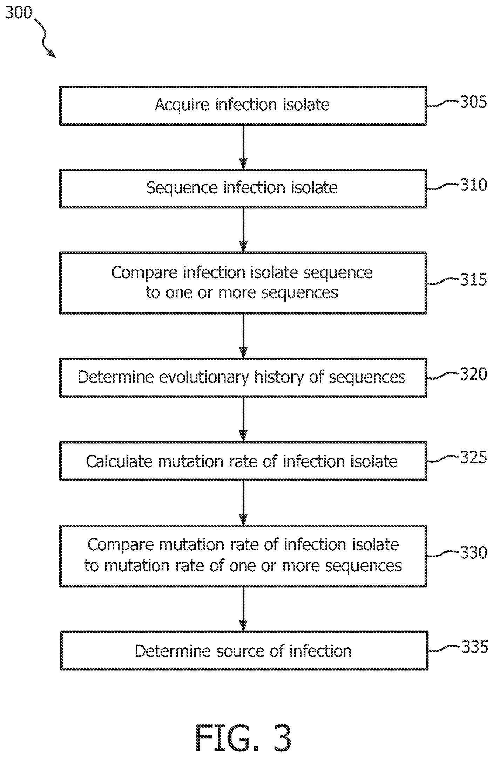

Figure 3 illustrates a flow chart of a method 300 of determining a source of an infection according to an embodiment of the disclosure. Medical staff may first collect samples from different environments to acquire an infection isolate or multiple infection isolates at Step 305. Samples may be collected routinely or may be collected in response to an identified HAI. The infection isolate may be processed and sequenced according to a sequencing technology known in the art at Step 310. Examples of companies that provide sequencing technology include 454 Life Sciences, a Roche company and Pacific Biosciences. Sequencing techniques known in the art may allow the entire genome of an infection isolate to be sequenced. A hospital may have sequencing technology on site or the hospital may send the samples to a separate sequencing company. A digital representation of the sequence may be generated and stored in a memory accessible to one or more processing units to allow analysis of the sequence. Unless otherwise noted, it will be assumed that any comparison, analysis, or determination based on a genetic sequence is performed by one or more processing units. The infection isolate sequence may then be compared to one or more sequences at Step 315 by the processing unit. The other sequences may be from other collected infection isolates, reference sequences of known pathogens from public or private databases, and/or sequences from other sources. The comparison may include determining variants between the infection isolate sequence and the one or more other sequences. Hereinafter, variants or genetic variants refer to single nucleotide differences or gene level differences. Variants may be found using existing software tools such as BWA-samstools and Golden Helix.

These variants may be used at Step 320 to determine the evolutionary history of the infection isolate sequence in relation to the one or more sequences. For example, it may be assumed that a given infectious agent exhibits single nucleotide mutations at a similar rate across all transmission domains, living or non-living. Consequently, a cumulative number of single nucleotide changes may be used to estimate a total number of sequence replication occurrences, i.e. total number of generations. As such, a further inference can be made that a variation in the evolutionary history, or the total number of generations may be attributed to the type of transmission domain. The evolutionary history may be determined by one of the methods described above or another method. Based on the evolutionary history, a rate of mutation of the infection isolate is calculated at Step 325. The rate of mutation may be calculated by one of the methods described above or another method.

According to one illustrative embodiment, the rate of mutation of the infection isolate is compared to the rate of mutation of one or more other sequences at Step 330. Based on the comparison of mutation rates, a determination of the source of infection is made in Step 335. The infection isolate sequence and other sequences may be clustered by mutation rates and each cluster assigned to a different infection source.

According to another aspect, not claimed herein, the rate of replication of the infection isolate is compared to the rate of replication of one or more other sequences at Step 330. Based on the comparison of replication rates, a determination of the source of infection is made in Step 335. The infection isolate sequence and other sequences may be clustered by replication rates and each cluster assigned to a different infection source. The infection isolate sequence may be compared to reference sequence replication rates from different known environments and assigned a source environment as will be described in more detail below. Other methods of determining infection source or categorization of infection sources of infection isolates may be performed.

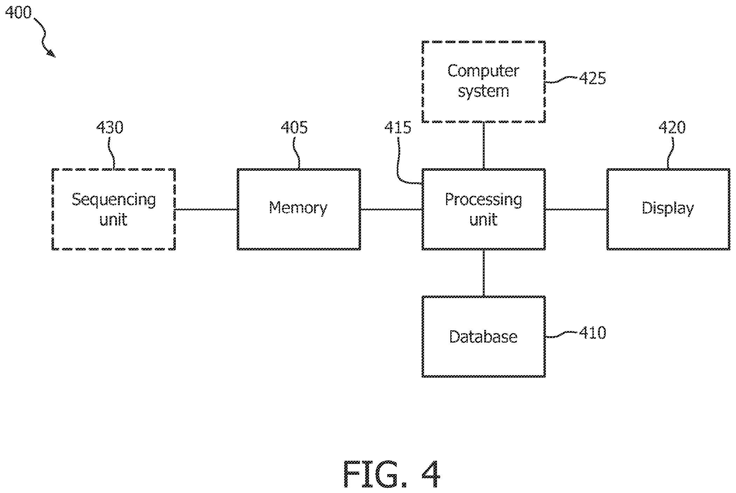

An example of a system 400 used for determining the source of transmission of infection according to an embodiment of the disclosure is shown as a block diagram in Figure 4. The infection isolate sequence in digital form may be included in memory 405. The memory 405 may be accessible to processing unit 415. The processing unit 415 may include one or more processing units. The processing unit 415 may have access to a database 410 that includes one or more sequences. The processing unit 415 may provide the results of its determination to a display 820 and/or the database 410. The display 420 may be an electronic display visible to a user. Optionally, processing unit 415 may further access a computer system 425. The computer system 425 may include additional databases, memories, and/or processing units. The computer system 425 may be a part of system 400 or remotely accessed by system 400. In some embodiments, the system 400 may also include a sequencing unit 430. The sequencing unit 430 may process the infection isolate to generate a sequence and produce the digital form of the infection isolate sequence.

Figures 2A-B illustrate an example according to an embodiment of the disclosure. In this example, 10 HAI samples may be collected from various reservoirs in similar or differing environments, and the entire genomes for the infection isolates may be sequenced. Variants may be determined and evolutionary distances may be calculated. Based on these evolutionary distances, an original tree 200 may be plotted as shown in Figure 2A. In this example, the original tree 200 was generated using the neighbor-joining tree method based on Kimura 2-parameter distance estimates. The tree 200 illustrates the evolutionary distances and relationships between the 10 HAIs. The length of the branches are proportional to these calculated distances. For example, node 11 and node 12, corresponding to two different HAIs, have an evolutionary distance of 0.72. As described previously, this is a measure of the similarity between the two HAIs. The greater the evolutionary distance, the lower the similarity between the sequences of the two HAIs. The relative ages in time for each node/HAI sample may be calculated based on original tree 200 of evolutionary distances by using a dating method, such as the MPL method described above. A dated tree 205 is shown in Figure 2B. In this example, in the original tree 100 the edge length from the 11th node to the 12th is 0.72 and from the 12th node to the node/leaf t10 is 0.99. If the age of a particular node is known, the rest of the nodes' ages may be estimated. In this example, the age of the 11th node is known, and is the common ancestor for all the other nodes of the tree. The MPL statistic for the 11th node is 1.56. Assuming node 11 diverged a minimum of two days ago, the rest of the nodes may be transformed to minimum ages in days by dividing by 0.78 = 1.56/2. For example, using this formula for age, node 12 diverged from node 11, 1.479 days ago.

Using this dating information it may be expected a sample may have a small number of mutations for a certain environment. If an evolutionary distance is high compared to the other samples or a reference sequence, and/or the calculated mutation rate or replication rate is higher compared to the other samples or the reference sequence, then the infection corresponding to that sample may be determined to be transmitted from an environment that allowed for a high replication rate of the pathogen. High replication rates may allow for a greater probability and occurrences for mutations, increasing differences between sequences of infection isolates collected at different points in time. For example, a sequence of a bacterium with a large evolutionary distance from another bacterial sequence may be determined to have been transmitted by a living organism. Living organisms may provide more nutrients and support higher replication rates for pathogens than non-living domains. Accordingly, infection isolates exhibiting an evolutionary distance, a cumulative mutation occurrences, a mutation rate, or a combination thereof, above or below a desired threshold value may be determined to be transmitted by a particular source.

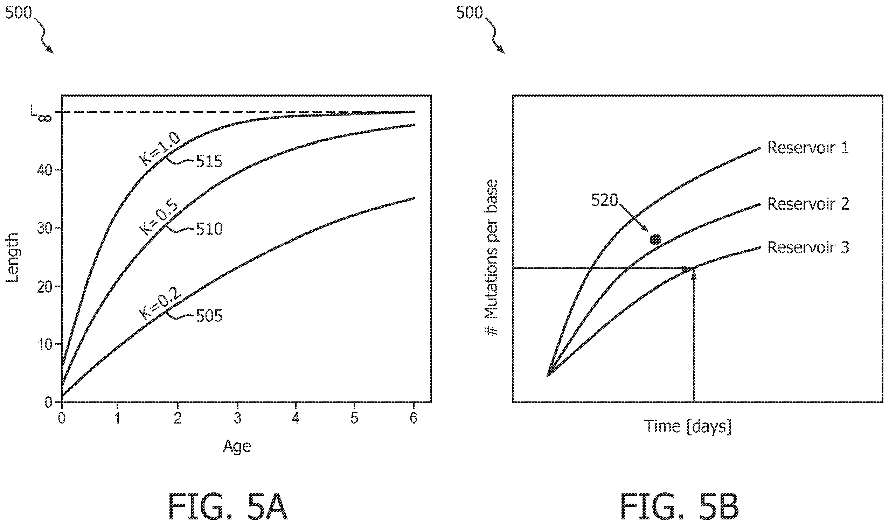

Alternatively or in addition to the methods used above, a replication or growth rate of a pathogen for one or more environments may be known. An example plot 500 of growth rates according to an embodiment of the disclosure is shown in Figure 5A. Based on the known growth rate, a particular number of mutations per generation may be expected.

According to one illustrative embodiment, referring to Figure 5B, a phylogenetically closest neighbor may be determined for a sample 520 under investigation by comparing the sequence of the sample with sequences of past samples stored in a database. Once a closest neighbor is identified, the number of differences (normalized to the total number of nucleotide bases compared) may be inferred. Under an assumption of a common mutation rate per replication cycle for this organism, characteristic mutation curves may be plotted for a plurality of reservoirs, (for example, reservoirs 1, 2 and 3 shown in Figure 5B). The mutation curves may be chosen based on the replication rates of this organism in different reservoirs, i.e. transmission domains. Based on a chronological time difference between the isolation of the sample under investigation and its determined nearest neighbor, an expected number of accumulated mutations is estimated for different growth rates. A growth rate that is closest to the actual, observed mutations may be identified as the growth rate of the sample, and a reservoir known to be associated with this growth rate may be identified as the most likely source of transmission (reservoir 2 for sample 520 in Figure 5B).

An infection isolate may be fitted to a growth rate curve based on its estimated growth rate. For example, if an infection isolate is found to have low growth rate (e.g., 0.2) and fits to curve 505, it may be determined the infection was transmitted by a surface. If the infection isolate is found to have a medium growth rate (e.g., 0.5) and fits to curve 510, it may be determined the infection was transmitted by a wet environment such as a toilet. If the infection isolate is found to have a high growth rate (e.g., 1.0) and fits to curve 515, the infection isolate may be determined to have been transmitted by a living organism.

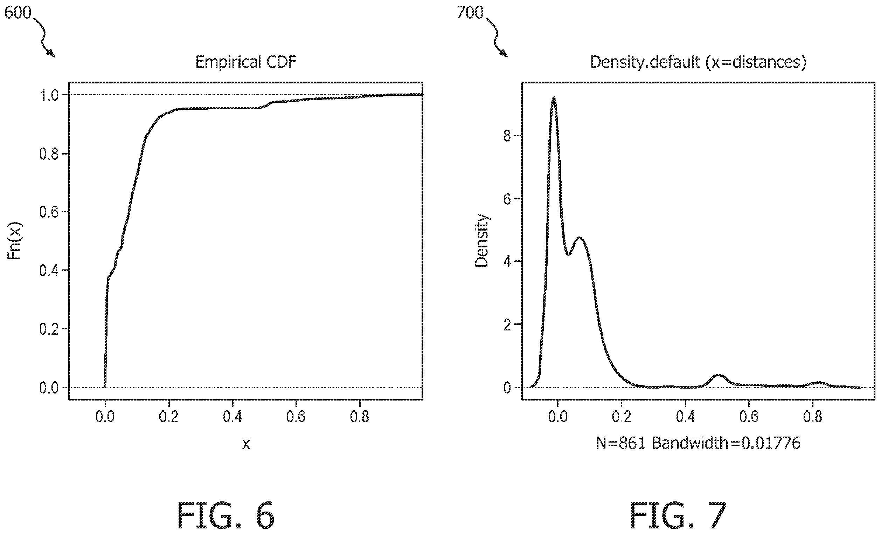

In addition or alternatively to the methods used above, the evolutionary distance distribution of infection isolate samples and/or reference sequences may be estimated. A new infection isolate sample's evolutionary distance may be compared to the evolutionary distances of known reference sequences or previously analyzed infection isolate samples. An empirical cumulative distribution function (ECDF) may be generated using known statistical methods. An example ECDF plot 600 according to an embodiment of the disclosure is shown in Figure 6. In this example, if an infection isolate was determined to fall outside a desired confidence interval (e.g., 95%), the infection isolate may be determined to have been transmitted from a different source than the reference sequences. In the plot shown in Figure 6, it may be determined that any infection isolate sequence having x >.8 has a high mutation rate compared to the reference sequences, and the infection was therefore transmitted by a living organism.

Figure 7 illustrates a different method according to an embodiment of the disclosure of clustering sequences based on evolutionary distances. Although represented in different graphical form, as shown in kernel density plot 700, the method is similar to the one described in reference to Figure 6. If an infection isolate sequence falls outside a desired confidence interval of evolutionary distances of the reference sequences, the infection isolate sequence is determined to have been transmitted by a different source than that of the reference sequences. In the plot 700, an infection isolate with an evolutionary distance of 0.2 or greater may be determined to have been transmitted by a living organism when relative age is low.

Although reference sequences are grouped into a single cluster in the examples shown in Figures 6 and 7, it may have multiple clusters of reference sequences. Each cluster may represent evolutionary distances for a pathogen in a particular environment. The confidence interval for a new infection isolate belonging to each cluster may then be calculated using known statistical methods. The source of transmission of an infection may then be determined based on which cluster the infection isolate sequence has the highest probability of inclusion.

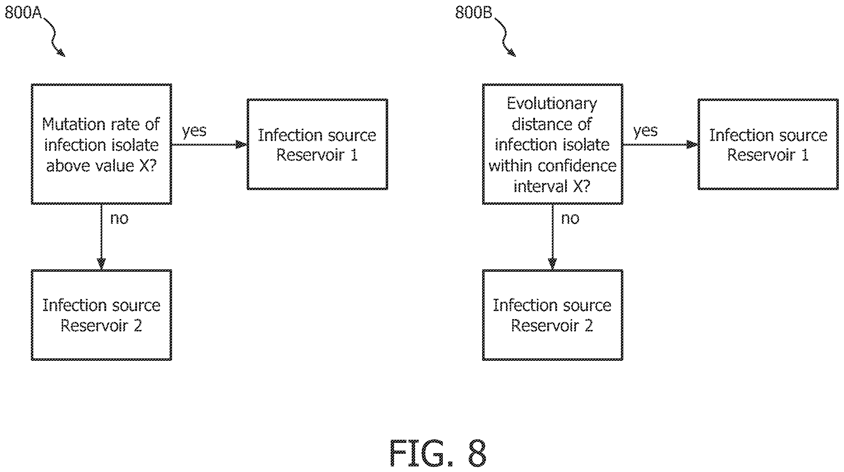

Figure 8 illustrates a flow chart that summarizes example processes 800A and 800B according to embodiments of the disclosure of determining the source of transmission of an infection. The processes 800A and/or 800B may be included in Step 335 of method 300 in Figure 3. Step 335 of method 300 may include one or more processes for determining the source of transmission of an infection. Process 800A determines whether the infection source is a first reservoir or a second reservoir based on whether the mutation rate of the infection isolate is greater or less than a desired threshold Value X. Process 800B determines whether the infection source is a first reservoir or a second reservoir based on whether the evolutionary distance of an infection isolate falls within a confidence interval X. The confidence interval may be based on a distribution of one or more sample sequences. Other processes may also be performed. Although only two reservoirs are shown, embodiments of the disclosure may include making a determination between any number of reservoirs. For example, in process 800A, Value X may be replaced by multiple values that define ranges of mutation rates. Each range may be associated with a different reservoir.

Once a determination of the source of transmission of an infection is made by the one or more processing units, based on the infection isolate sequence stored in memory, the determination may be provided by the at least one processing unit to a user, a database, and/or computer system. A user may be a member of the infection control staff at a hospital. The user may receive a visual indicator on an electronic display coupled to the processing unit. The user may then use the determination of the infection source to coordinate efforts by the infection control staff to take measures to isolate and remove the source of infection. For example, if the infection source is determined to be from a living organism, such as a particular patient, the infection control staff may isolate a patient and ensure nurses and doctors use elevated sterilization methods after treating the patient before contacting other patients. If the infection source is determined to be from a non-living domain, such as a bronchoscope, the equipment may be sterilized and retested for pathogens before being used in additional patients.

Multiple infection isolates may be determined to all be from different unrelated sources. In these instances, infection control staff may determine that sterile procedures and/or infection control protocols have broken down at the health care facility. Further analysis and/or retraining of medical staff of proper procedures to reduce transmission of infection may be executed.

The determination and infection isolate sequence may be stored in a database for use in future infection source determinations. The data may also be transferred to a computer system accessible by additional users, hospitals, or agencies. This may allow for easier information sharing and improve infection control. It may also allow for easier reporting of infections, which may be required by regulations. For example, the processing unit may automatically generate a report with the determination and provide the report to an infectious disease tracking agency such as the U.S. Center for Disease Control (CDC).

Of course, it is to be appreciated that any one of the above embodiments or processes may be combined with one or more other embodiments and/or processes or be separated and/or performed amongst separate devices or device portions in accordance with the present systems, devices and methods.

**Claims**

1. A method, comprising:

   receiving an electronic data representative of an infection isolate sequence;
   comparing (315) the electronic data representative of the infection isolate sequence to at least one reference sequence stored in a database to determine variants between the infection isolate sequence and the at least one reference sequence;
   determining (320) at least a portion of an evolutionary history of the infection isolate based, at least in part, on the variants;
   calculating, (325) based at least in part on the portion of the evolutionary history, a mutation rate of the infection isolate, wherein the mutation rate is calculated by the Mean Path Lengths method;
   comparing (330) the mutation rate of the infection isolate to a mutation rate of the at least one reference sequence;
   determining (335), based on a difference between the mutation rate of the infection isolate and the mutation rate of the at least one reference sequence, whether the infection isolate was transmitted by a first reservoir or a second reservoir, wherein each at least one reference sequence is associated with one of the first and second reservoirs; and

providing an indication of whether the infection isolate was transmitted by the first reservoir or second reservoir.

2. The method of claim 1, wherein the indication provided is for quarantining the first reservoir or second reservoir based, at least in part, on the determination of whether the infection isolate was transmitted by the first reservoir or second reservoir.

3. The method of claim 1, wherein the indication is text on an electronic display.

4. The method of claim 1, wherein determining at least a portion of the evolutionary history includes calculating an evolutionary distance.

5. The method of claim 4, wherein the evolutionary distance is calculated by the Jukes-Cantor Method.

6. The method of claim 1, wherein the first reservoir is a living organism and the second reservoir is a non-living domain.

7. The method of claim 6, further comprising providing a notice to the user to perform any one of the following:

disinfect a piece of equipment if the determination is the infection isolate was transmitted by the non-living domain;
test a piece of equipment for the infection isolate if the determination is the infection isolate was transmitted by the non-living domain;
test a patient for the infection isolate if the determination is the infection isolate was transmitted by the living organism; and
quarantine a patient if the determination is the infection isolate was transmitted by the living organism.

8. The method of claim 1, further comprising generating a report of the determination of whether the infection isolate was transmitted by the first reservoir or second reservoir for sending to an infection tracking agency.

9. A system (400), comprising:

a processing unit (415);
a memory (405) accessible to the processing unit;
a database (410) accessible to the processing unit; and
a display (420) coupled to the processing unit;
wherein the processing unit is configured to:

compare a sequence of an infection isolate stored in the memory to at least one reference sequence stored in the database to determine variants between the infection isolate sequence and the at least one reference sequence;
determining at least a portion of an evolutionary history of the infection isolate based, at least in part, on the variants;
calculating, based at least in part on the portion of the evolutionary history, a mutation rate of the infection isolate, wherein the mutation rate is calculated by the Mean Path Lengths method;
compare the mutation rate of the infection isolate to a mutation rate of the at least one reference sequence
determining whether the infection isolate was transmitted by a first reservoir or a second reservoir, based on a difference of the mutation rate of the infection isolate and the mutation rate of the at least one reference sequence, wherein each at least one reference sequence is associated with one of the first and second reservoirs; and
provide to the display the determination of whether the infection isolate was transmitted by the first reservoir or second reservoir.

10. The system of claim 9, further comprising a computer system accessible to the processing unit, wherein the processing unit is configured to provide the determination of whether the infection isolate was transmitted by the first reservoir or second reservoir.

11. The system of claim 9, further comprising a sequencing unit (430) configured to provide the sequence of the infection isolate to the memory.

12. The system of claim 9, wherein the processing unit comprises a plurality of processing units.

**Patentansprüche**

1. Verfahren, umfassend:

   Empfangen elektronischer Daten, die repräsentativ für eine Infektionsisolatsequenz sind; Vergleichen (315) der elektronischen Daten, die repräsentativ für eine Infektionsisolatsequenz sind, mit mindestens einer in einer Datenbank gespeicherten Referenzsequenz, um Varianten zwischen der Infektionsisolatsequenz und der mindestens einen Referenzsequenz zu bestimmen;
   Bestimmen (320) mindestens eines Abschnitts einer Evolutionsgeschichte des Infektionsisolats, zumindest teilweise basierend auf den Varianten;
   Berechnen (325), zumindest teilweise basierend auf dem Abschnitt der Evolutionsgeschichte, einer Mutationsrate des Infektionsisolats, wobei die Mutationsrate durch das Verfahren der mittleren Weglängen berechnet wird;
   Vergleichen (330) der Mutationsrate des Infektionsisolats mit einer Mutationsrate der mindestens einen Referenzsequenz;
   Bestimmen (335), basierend auf einer Differenz zwischen der Mutationsrate des Infektionsisolats und der Mutationsrate der mindestens einen Referenzsequenz, ob das Infektionsisolat durch ein erstes Reservoir oder ein zweites Reservoir übertragen wurde, wobei jede mindestens eine Referenzsequenz mit einem von dem ersten und dem zweiten Reservoir assoziiert ist; und
   Bereitstellen einer Angabe, ob das Infektionsisolat durch das erste Reservoir oder das zweite Reservoir übertragen wurde.

2. Verfahren nach Anspruch 1, wobei die bereitgestellte Angabe zur Quarantäne des ersten Reservoirs oder des zweiten Reservoirs zumindest teilweise auf der Bestimmung basiert, ob das Infektionsisolat durch das erste Reservoir oder das zweite Reservoir übertragen wurde.

3. Verfahren nach Anspruch 1, wobei die Angabe Text auf einer elektronischen Anzeige ist.

4. Verfahren nach Anspruch 1, wobei ein Bestimmen zumindest eines Abschnitts der Evolutionsgeschichte ein Berechnen eines Evolutionsabstands beinhaltet.

5. Verfahren nach Anspruch 4, wobei der evolutionäre Abstand durch das Jukes-Cantor-Verfahren berechnet wird.

6. Verfahren nach Anspruch 1, wobei das erste Reservoir ein lebender Organismus und das zweite Reservoir eine nicht lebende Domäne ist.

7. Verfahren nach Anspruch 6, ferner umfassend ein Bereitstellen eines Hinweises an den Benutzer, ein beliebiges von Folgenden auszuführen:

   Desinfizieren eines Ausrüstungsteils, wenn die Bestimmung derart ist, dass das Infektionsisolat durch die nicht lebende Domäne übertragen wurde;
   Testen eines Ausrüstungsteils auf das Infektionsisolat, wenn die Bestimmung derart ist, dass das Infektionsisolat durch die nicht lebende Domäne übertragen wurde;
   Testen eines Patienten auf das Infektionsisolat, wenn die Bestimmung derart ist, dass das Infektionsisolat durch den lebenden Organismus übertragen wurde; und
   Stellen eines Patienten unter Quarantäne, wenn die Bestimmung derart ist, dass das Infektionsisolat durch den lebenden Organismus übertragen wurde.

8. Verfahren nach Anspruch 1, ferner umfassend ein Erstellen eines Berichts über die Bestimmung, ob das Infektionsisolat durch das erste Reservoir oder das zweite Reservoir übertragen wurde, zum Senden an eine Infektionsverfolgungsbehörde.

9. System (400), umfassend:

   eine Verarbeitungseinheit (415);
   einen Speicher (405), der für die Verarbeitungseinheit zugänglich ist;

eine Datenbank (410), die für die Verarbeitungseinheit zugänglich ist; und

eine Anzeige (420), die mit der Verarbeitungseinheit gekoppelt ist;

wobei die Verarbeitungseinheit zu Folgendem konfiguriert ist:

vergleichen einer in dem Speicher gespeicherten Sequenz eines Infektionsisolats mit mindestens einer in der Datenbank gespeicherten Referenzsequenz, um Varianten zwischen der Infektionsisolatsequenz und der mindestens einen Referenzsequenz zu bestimmen;

Bestimmen mindestens eines Abschnitts der Evolutionsgeschichte des Infektionsisolats, zumindest teilweise basierend auf den Varianten;

Berechnen, zumindest teilweise basierend auf dem Abschnitt der Evolutionsgeschichte, einer Mutationsrate des Infektionsisolats, wobei die Mutationsrate durch das Verfahren der mittleren Weglängen berechnet wird;

Vergleichen der Mutationsrate des Infektionsisolats mit einer Mutationsrate der mindestens einen Referenzsequenz;

Bestimmen, ob das Infektionsisolat durch ein erstes Reservoir oder ein zweites Reservoir übertragen wurde, basierend auf einer Differenz der Mutationsrate des Infektionsisolats und der Mutationsrate der mindestens einen Referenzsequenz, wobei jede mindestens eine Referenzsequenz mit einem von dem ersten und dem zweiten Reservoir assoziiert ist; und

Bereitstellen der Bestimmung an die Anzeige, ob das Infektionsisolat durch das erste Reservoir oder das zweite Reservoir übertragen wurde.

10. System nach Anspruch 9, ferner umfassend ein Computersystem, das für die Verarbeitungseinheit zugänglich ist, wobei die Verarbeitungseinheit konfiguriert ist, um die Bestimmung bereitzustellen, ob das Infektionsisolat durch das erste Reservoir oder das zweite Reservoir übertragen wurde.

11. System nach Anspruch 9, ferner umfassend eine Sequenziereinheit (430), die konfiguriert ist, um die Sequenz des Infektionsisolats an den Speicher bereitzustellen.

12. System nach Anspruch 9, wobei die Verarbeitungseinheit eine Vielzahl von Verarbeitungseinheiten umfasst.

**Revendications**

1. Méthode comprenant les étapes consistant à:

recevoir des données électroniques représentatives d'une séquence d'isolats d'infection; comparer (315) les données électroniques représentatives de la séquence d'isolats d'infection à au moins une séquence de référence stockée dans une base de données afin de déterminer les variantes entre la séquence d'isolats d'infection et au moins une séquence de référence;

déterminer (320) au moins une partie de l'histoire évolutive des isolats d'infection sur la base, au moins en partie, des variantes;

calculer, (325) en se basant au moins en partie sur la partie de l'histoire évolutive, un taux de mutation de l'isolat d'infection, le taux de mutation étant calculé par la méthode « Mean Path Lengths »; comparer (330) le taux de mutation de l'isolat d'infection à un taux de mutation de la séquence de référence au moins;

déterminer (335), sur la base d'une différence entre le taux de mutation de l'isolat d'infection et le taux de mutation de l'au moins une séquence de référence, si l'isolat d'infection a été transmis par un premier ou un deuxième réservoir, chaque au moins une séquence de référence étant associée à l'un du premier ou du deuxième réservoir; et indiquer si l'isolat infectieux a été transmis par le premier ou le deuxième réservoir.

2. Méthode selon la revendication 1, dans laquelle l'indication fournie est pour la mise en quarantaine du premier ou du deuxième réservoir sur la base, au moins en partie, de la détermination de la transmission de l'isolat d'infection par le premier ou le deuxième réservoir.

3. Méthode selon la revendication 1, dans laquelle l'indication est un texte sur un écran électronique.

4. Méthode selon la revendication 1, dans laquelle la détermination d'au moins une partie de l'histoire évolutive comprend le calcul d'une distance évolutive.

5. Méthode selon la revendication 4, dans laquelle la distance évolutive est calculée par la méthode Jukes-Cantor.

**6.** Méthode selon la revendication 1, dans laquelle le premier réservoir est un organisme vivant et le deuxième un domaine non vivant.

**7.** Méthode selon la revendication 6, comprenant en outre la fourniture d'un avis à l'utilisateur pour qu'il effectue l'une des opérations suivantes:

désinfecter une pièce d'équipement s'il est déterminé que l'isolat d'infection a été transmis par le domaine non vivant;

tester une pièce d'équipement pour détecter l'isolat d'infection si l'on détermine que l'isolat d'infection a été transmis par le domaine non vivant;

tester un patient pour l'isolat d'infection si la détermination est que l'isolat d'infection a été transmis par l'organisme vivant; et

mettre un patient en quarantaine s'il est établi que l'isolat d'infection a été transmis par l'organisme vivant.

**8.** Méthode selon la revendication 1 comprend en outre la production d'un rapport sur la détermination de la transmission de l'isolat d'infection par le premier ou le deuxième réservoir, qui sera envoyé à un organisme de suivi des infections.

**9.** Système (400) comprenant

une unité de traitement (415);

une mémoire (405) accessible à l'unité de traitement; une base de données (410) accessible à l'unité de traitement; et

un écran (420) couplé à l'unité de traitement; dans lequel l'unité de traitement est configurée pour:

comparer une séquence d'un isolat d'infection stockée dans la mémoire à au moins une séquence de référence stockée dans la base de données pour déterminer les variantes entre la séquence de l'isolat d'infection et

au moins une séquence de référence;

déterminer au moins une partie de l'histoire évolutive de l'isolat d'infection en se basant, au moins en partie, sur les variantes;

calculer, en se fondant au moins en partie sur la partie de l'histoire évolutive, un taux de mutation de l'isolat d'infection, le taux de mutation étant calculé par la méthode « Mean Path Lengths »;

comparer le taux de mutation de l'isolat d'infection à un taux de mutation de la séquence de référence au moins: déterminer si l'isolat d'infection a été transmis par un premier ou un deuxième réservoir, sur la base d'une différence entre le taux de mutation de l'isolat d'infection et le taux de mutation de la séquence de référence au moins, chaque séquence de référence au moins étant associée à l'un des deux réservoirs;

et fournir à l'écran la détermination de la transmission de l'isolat d'infection par le premier ou le deuxième réservoir.

**10.** Système selon la revendication 9 comprend en outre un système informatique accessible à l'unité de traitement, dans lequel l'unité de traitement est configurée pour déterminer si l'isolat d'infection a été transmis par le premier ou le deuxième réservoir.

**11.** Système selon la revendication 9 comprend en outre une unité de séquençage (430) configurée pour fournir la séquence de l'isolat d'infection à la mémoire.

**12.** Système selon la revendication 9, dans lequel l'unité de traitement comprend une pluralité d'unités de traitement.

FIG. 1

Original tree - 200

The dated MPL tree - 205

FIG. 2A

FIG. 2B

EP 3 180 722 B1

300

| Acquire infection isolate | 305 |
| Sequence infection isolate | 310 |
| Compare infection isolate sequence to one or more sequences | 315 |
| Determine evolutionary history of sequences | 320 |
| Calculate mutation rate of infection isolate | 325 |
| Compare mutation rate of infection isolate to mutation rate of one or more sequences | 330 |
| Determine source of infection | 335 |

FIG. 3

FIG. 4

EP 3 180 722 B1

FIG. 5B

FIG. 5A

FIG. 6

FIG. 7

EP 3 180 722 B1

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2002120408 A1

### Non-patent literature cited in the description

- **MAGILL SS ; EDWARDS JR ; BAMBERG W et al.** Multistate Point-Prevalence Survey of Health Care-Associated Infections. *N Engl J Med,* 2014, vol. 370, 1198-208
- **SCOTT II, R. D.** The Direct Medical Costs of Healthcare-Associated Infections in U.S. Hospitals and the Benefits of Prevention. *CDC,* March 2009
- **RAGHEB M N et al.** The mutation rate of mycobacterial repetitive unit loci in strains of M. tuberculosis from cynomolgus macaque infection. *BMC Genomics,* 05 March 2013, vol. 14 (1
- **FORD C B et al.** Mycobacterium tuberculosis mutation rate estimates from different lineages predict substantial differences in the emergence of drug-resistant tuberculosis. *Nature Genetics,* 01 January 2013, vol. 45 (7
- **T. H. JUKES ; C. R. CANTOR.** Evolution of protein molecules In Mammalian protein metabolism. 1969, vol. III, 21-132
- **KIMURA, MOTOO.** A simple method for estimating evolutionary rates of base substitutions through comparative studies of nucleotide sequences. *Journal of molecular evolution,* 1980, vol. 16 (2), 111-120
- **SAITOU N ; NEI M.** The neighbor-joining method: a new method for reconstructing phylogenetic trees. *Molecular Biology and Evolution,* July 1987, vol. 4 (4), 406-425
- **BRITTON ; TOM et al.** Phylogenetic dating with confidence intervals using mean path lengths. *Molecular phylogenetics and evolution,* 2002, vol. 24 (1), 58-65